(19)

Europäisches
Patentamt
European
Patent Office
Office européen
des brevets

(11) **EP 4 345 829 A1**

(12) **EUROPEAN PATENT APPLICATION**

(43) Date of publication:
**03.04.2024  Bulletin 2024/14**

(21) Application number: **22197988.3**

(22) Date of filing: **27.09.2022**

(51) International Patent Classification (IPC):
**G16H 20/40** (2018.01)   **G16H 30/40** (2018.01)
**G16H 50/50** (2018.01)

(52) Cooperative Patent Classification (CPC):
**G16H 20/40; G16H 30/40; G16H 50/50;**
G16H 50/20; G16H 70/20

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Siemens Healthineers AG**
  **91301 Forchheim (DE)**
• **Friedrich-Alexander-Universität**
  **Erlangen-Nürnberg**
  **91054 Erlangen (DE)**

(72) Inventors:
• **Audigier, Chloé**
  **3006 Bern (CH)**

• **Lluch Alvarez, Eric**
  **90419 Nürnberg (DE)**
• **Meister, Felix**
  **91058 Erlangen (DE)**
• **Mihalef, Viorel**
  **North Brunswick, 08902 (US)**
• **Passerini, Tiziano**
  **Plainsboro, 08536 (US)**

(74) Representative: **Siemens Healthineers
Patent Attorneys
Siemens Healthcare GmbH
SHS TE IP
Henkestraße 127
91052 Erlangen (DE)**

Remarks:
Amended claims in accordance with Rule 137(2)
EPC.

(54) **COMPUTER-IMPLEMENTED SOFT TISSUE EMULATION SYSTEM AND METHOD**

(57)   A soft tissue emulation system, comprising: an input interface, configured to obtain imaging data of the soft tissue; a computing unit, configured to implement an artificial neural network, which is adapted to generate, using the obtained imaging data as input, and a biophysical model of the soft tissue, a digital twin of the soft tissue at different times, wherein the biophysical model describes the response of the soft tissue to thermal and/or electromechanical stimuli over time, and wherein the generation of the digital twin at one time is independent of the generation of the digital twin at another time; and an output interface, configured to output a representation of the soft tissue over time based on the digital twin generated by the artificial neural network.

FIG 1

EP 4 345 829 A1

**Description**

The present invention relates to a soft tissue emulation system for medical applications, especially for living mammalian organs. In particular, it relates to the use of artificial neural networks to infer the behaviour of the soft tissue under external stimuli.

The invention is mostly described with respect to cardiac tissue and liver tissue in humans, but the principles of the invention have a broader scope.

The simulation and emulation of the behaviour of human organs under stimulation of their tissue with external stimuli (e.g., electric pulses or heat) is a technology that is under constant development. A reliable description of the reaction of human tissue to externally applied stimulation can improve the treatment of certain diseases or conditions.

In cardiology, in particular, the description of the cardiac tissue can be simulated through biomechanical models, which are based on differential equations describing the evolution of the cardiac tissue with time under, e.g., electrical pulses. Cardiac computational models compute solutions to mathematical equations that combine in-depth insights of physiology and physics. When properly personalized to the patient's anatomy and physiology, these models allow for a forecasting of treatment outcomes and therefore can assist in choosing an optimal patient-specific treatment.

Different solutions to simulate a patient's electrophysiology have been proposed over the last decades. However, the use of electromechanical modeling (i.e. simulating the highly non-linear and anisotropic biomechanical behavior of cardiac tissue under electrical stimulation) is still hindered by the simulation speed of the numerical solvers, which seldom meet the time or accuracy requirements for the translation into the clinical environment.

Artificial intelligence (AI), and in particular the use of deep learning, is a promising way to improve the speed of the numerical solvers. The state-of-the-art implementation of AI-aided methods is however still far from a real-time simulation of human tissue.

It is an object of the present invention to provide a computer-implemented soft tissue emulation system that can infer the response of soft tissue to external stimuli in real-time.

The purpose of this invention is achieved through a system with the features disclosed in claim 1 and a method with the features detailed in claim 12. Preferred embodiments of the invention with advantageous features are given in the dependent claims.

A first aspect of the invention provides a soft tissue emulation system, comprising: an input interface, configured to obtain imaging data of the soft tissue; a computing unit, configured to implement an artificial neural network, which is adapted to generate, using the obtained imaging data as input and a biophysical model of the soft tissue, a digital twin of the soft tissue at different times, wherein the biophysical model describes the response of the soft tissue to thermal and/or electromechanical stimuli over time, and wherein the generation of the digital twin at one time is independent of the generation of the digital twin (or, more precisely, its state) at another time; and an output interface, configured to output a representation of the soft tissue over time based on the digital twin generated by the artificial neural network.

The soft tissue may in particular be cardiac tissue or hepatic tissue.

The input interface and/or the computing unit and/or the output interface are broadly understood as entities capable of acquiring, obtaining, receiving or retrieving image data and delivering it for further processing. Each of them, or parts thereof, may therefore contain, at least, a central processing unit, CPU, and/or at least one graphics processing unit, GPU, and/or at least one field-programmable gate array, FPGA, and/or at least one application-specific integrated circuit, ASIC and/or any combination of the foregoing. Each of them may further comprise a working memory operatively connected to the at least one CPU and/or a non-transitory memory operatively connected to the at least one CPU and/or the working memory. Each of them may be implemented partially and/or completely in a local apparatus and/or partially and/or completely in a remote system such as by a cloud computing platform.

All of the elements of the emulation system may be realized in hardware and/or software, cable-bound and/or wireless, and in any combination thereof. Any of the elements may comprise an interface to an intranet or the Internet, to a cloud computing service, to a remote server and/or the like.

In particular, the emulation system may be implemented partially and/or completely in a local apparatus, e.g. a computer, in a system of computers and/or partially and/or completely in a remote system such as a cloud computing platform.

In systems based on cloud computing technology, a large number of devices is connected to a cloud computing system via the Internet. The devices may be located in a remote facility connected to the cloud computing system. For example, the devices can comprise, or consist of, equipments, sensors, actuators, robots, and/or machinery in an industrial set-up(s). The devices can be medical devices and equipments in a healthcare unit. The devices can be home appliances or office appliances in a residential/commercial establishment.

The cloud computing system may enable remote configuring, monitoring, controlling, and maintaining connected devices (also commonly known as 'assets'). Also, the cloud computing system may facilitate storing large amounts of data periodically gathered from the devices, analyzing the large amounts of data, and providing insights (e.g., Key Performance Indicators, Outliers) and alerts to operators, field engineers or owners of the devices via a graphical user interface (e.g., of web applications). The insights and alerts may enable controlling and maintaining the devices, leading to efficient and fail-safe operation of the devices. The cloud computing system may also enable modifying parameters associated with the devices and issues control commands via the graphical user interface based on the insights and alerts.

The cloud computing system may comprise a plurality of servers or processors (also known as 'cloud infrastructure'), which are geographically distributed and connected to each other via a network. A dedicated platform (hereinafter referred to as 'cloud computing platform') is installed on the servers/processors for providing above functionality as a service (hereinafter referred to as 'cloud service'). The cloud computing platform may comprise a plurality of software programs executed on one or more servers or processors of the cloud computing system to enable delivery of the requested service to the devices and its users.

One or more application programming interfaces (APIs) are deployed in the cloud computing system to deliver various cloud services to the users.

Imaging data broadly describes any information that can be used for further processing and analysis in medical applications. In the context of the present invention, the imaging data may consist of one or more scanned images of soft tissue samples of organs of a mammal, e.g. CT images or MRI images. Some of these images may comprise a selected sub-image portion or a crop of a larger image, where segmentation or tessellation has been applied. Imaging data can also comprise metadata e.g. in the form of annotations.

The computing unit comprises an artificial neural network ANN. Whenever herein an artificial neural network is mentioned, it shall be understood as a computerized entity able to implement different data analysis methods broadly described under the terms artificial intelligence, machine learning, deep learning or computer learning. The artificial neural network can be a generative adversarial network (GAN), a convolutional neural network (CNN) or any other neural network or combination thereof.

A second aspect of the present invention provides a computer-implemented soft tissue emulation method, comprising the following steps: (a) obtaining imaging data of a soft tissue; (b) generating, using a biophysical model of the soft tissue and an artificial neural network configured and trained to receive the acquired imaging data as input, a digital twin of the soft tissue at different times, wherein the biophysical model simulates the response of the soft tissue to thermal and/or electromechanical stimuli over time, and wherein the generating of the digital twin at one time is independent of the generating of the digital twin at another time; and (c) outputting a representation of the soft tissue over time based on the digital twin generated at the different times.

In particular, the method according to the second aspect of the invention may be carried out by the system according to the first aspect of the invention. The features and advantages disclosed herein in connection with the energy management system are therefore also disclosed for the method, and vice versa.

According to a third aspect, the invention provides a computer program product comprising executable program code configured to, when executed, perform the method according to the second aspect of the present invention.

According to a fourth aspect, the invention provides a non-transient computer-readable data storage medium comprising executable program code configured to, when executed, perform the method according to the second aspect of the present invention.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

According to a fifth aspect, the invention provides a data stream comprising, or configured to generate, executable program code configured to, when executed, perform the method according to the second aspect of the present invention.

One of the main ideas underlying the present invention is to provide a computer-implemented soft tissue emulation system, in particular for application to the emulation of cardiac tissue using an artificial neural network, ANN, which is adapted to use non-autoregressive inference as a solver for a biophysical model that describes the response of cardiac tissue to electrical stimuli. The emulation system is configured to obtain imaging data of cardiac tissue (comprising e.g. MRI images) and feed them into the ANN, trained and configured to generate a digital twin of the soft tissue at different times based on the solution of the biophysical model. These digital twins can be used to obtain a representation or a simulation of the movement of the cardiac tissue over time as a response to electrical stimulation.

The device as described above allows a simple implementation of a computer-implemented soft tissue emulation method, in particular for cardiac tissue emulation. In a first step, imaging data of cardiac tissue is obtained. This data is used as input to a biophysical model describing the response of cardiac tissue to electrical stimulation. In another step a digital twin of the cardiac tissue is generated at different times, under the use of an ANN, which is trained and configured to solve the biophysical model at different times. In a further step a representation of the cardiac tissue over time is output, taking into account the digital twin generated at different times.

One advantage of the present invention is that using an ANN with non-autoregressive inference allows an emulation of soft tissue in real time. This is particularly interesting for cardiac tissue simulation, since it allows to incorporate the simulation of the heart into a clinical and even a surgical environment, where the surgeon could visualize the expected reactions of the heart to different stimuli and thus obtain valuable information to get assistance in decision making, both in the planning of a surgical intervention (preoperative stage) as well as in the intraoperative stage. This is an improvement over conventional methods, which are much slower and cannot be used in real time.

Another advantage of the present invention is that the accuracy of the solutions only depends on the capacity of the ANN and the quality of the imaging data used in its training. The non-autoregressive inference solves the biophysical model in a non-sequential manner, i.e. the solution at a particular time does not depend on the solution at a different (normally previous) time. Numerical uncertainties of the solution at a certain time do not have an impact (especially do not accumulate or do not get propagated) at a different time. One particularly interesting consequence is that the emulation system can emulate the end-diastolic and end-systolic heart shape fast and independently. The end-systolic and end-diastolic times are particularly useful to obtain information about the volumes of blood prior to and right after a heartbeat. These volumes are used as benchmarks to learn whether a patient has a heart condition or anomaly.

A further advantage of the present invention is that it can be installed in a handheld device, for instance a tablet or a cell phone, which facilitates its implementation in clinical and surgical environments. Non-autoregressive inference as used in the invention requires a rather modest demand on computing power in order to provide reliable emulations of the dynamical behavior of cardiac tissue.

Yet another advantage of the invention is that it can be used with large volumes of data. Since non-autoregressive inference is not sequential, the emulation lends itself to parallel processing, which can lead to fast performances even when the amount of data is sizeable. This also enables patient-specific data processing.

Advantageous embodiments and further developments follow from the dependent claims as well as from the description of the different preferred embodiments illustrated in the accompanying figures.

According to some embodiments, refinements, or variants of embodiments, the artificial neural network comprises a branch network module, which implements a first convolutional neural network configured to infer structural information of the soft tissue, and a trunk network module, which implements a second convolutional neural network configured to generate a solution to the biophysical model at different times.

One variant of a mathematical model for the time evolution of soft tissue using non-autoregressive inference divides the ANN into two convolutional neural networks CNN, one which infers the geometric aspects of the cardiac tissue (i.e. information on variables related to the shape of the cardiac tissue) and another one which contains time samples where the soft tissue is to be emulated. The two parts are combined to deliver a digital twin of the soft tissue at the sample times.

According to some embodiments, refinements, or variants of embodiments, the soft tissue emulation system is configured to emulate the electromechanical properties of cardiac tissue. As already emphasized in the foregoing, one of the main applications of the present invention is the emulation of cardiac tissue under electrical stimulation in real time.

According to some embodiments, refinements, or variants of embodiments, the biophysical model is based on a second order ordinary differential equation describing the deformation of cardiac tissue in response to at least one electrophysiological information. The biophysical model for cardiac tissue boils down to an elastodynamics equation, which describes the shape evolution of the heart over time under space- and time-dependent electrical stimulation, through a differential equation that involves the instantaneous displacement, velocity and acceleration of the cardiac tissue.

According to some embodiments, refinements, or variants of embodiments, the electrophysiological information comprises a set of local activation times and/or a sequence of electric potentials. An electrical stimulation can be parametrized with either of the two sets of variables or a combination thereof. They comprise information about the space and time dependence of the electrical stimuli applied onto the cardiac tissue.

According to some embodiments, refinements, or variants of embodiments, the structural information of the soft tissue comprises a tetrahedral mesh, with each vertex characterized by a set of local cylindrical coordinates and/or a distance between endocardium and epicardium, and/or an indication whether a vertex belongs to the endocardium or to the epicardium. Providing a tetrahedral mesh is a way of discretizing a volume, in this case the heart or part of the cardiac tissue. Describing the 3D vertex coordinates of the mesh with a local cylindrical coordinate system (radius, angle, height) has the advantage that it conveys an axial symmetry that the ANN is trained to learn. This way the inference is robust against rotations and the amount of training data can be reduced.

The local coordinate system can be supplemented with global quantities, e.g. the distance from endocardium to epicardium. In addition, one may denote special vertices using categorical features. For instance, one may label vertices as belonging to the endocardium or the epicardium in order to better model the boundary conditions. These boundary conditions could be the blood pressure force on the endocardium and/or the external force on the epicardium, which describes also its interaction with the pericardium. Tests performed with the system of the invention have shown that these annotations contribute to a more efficient inference of structural information by the ANN.

According to some embodiments, refinements, or variants of embodiments, the soft tissue emulation system is configured to emulate the local temperature distribution of the soft tissue when stimulated by an external heat source. This is of special relevance, e.g., for the treatment of liver cancers, i.e. when the soft tissue is hepatic tissue. A minimally invasive procedure when tumors have nodules smaller than 2-3cm is radio frequency ablation (RFA). In an RFA procedure, heat (i.e. strong electric fields) is applied to the tumor areas through one or more percutaneously inserted electrodes. The emulation system described in the present invention enables patient-specific planning for RFA procedures which can be generated in real-time. Another application where these features are of relevance is in the context of ventricular ablation of the heart, i.e. when the soft tissue is cardiac tissue.

According to some embodiments, refinements, or variants of embodiments, the structural information of the soft tissue is characterized by at least one of the following physiological properties: density, heat conductivity, location of the blood vessels and blood flow rate. One relevant example is, e.g., when the soft tissue is hepatic tissue. In order to plan an RFA, an advection-diffusion equation may be solved. This equation comprises information about the geometric characteristics of the hepatic tissue. Especially important is the irrigation and perfusion of the liver (i.e. precise knowledge of the position of blood vessels and the blood flow), together with the response of the liver to heat (parametrized through the heat conductivity). Similarly the soft tissue may be cardiac tissue.

According to some embodiments, refinements, or variants of embodiments, the imaging data comprises information about the presence of tumors in the hepatic tissue, and the computing unit further comprises an electrode module configured to use the information about the presence of tumors in the hepatic tissue as input, and generate as output a corresponding electrode configuration for an ablation planning, characterized at least by the location of the electrodes, their radius, and/or the time during which they are active. With the emulation system of the invention the optimal ablation zones for an RFA planning can be identified based on patient-specific characteristics.

According to some embodiments, refinements, or variants of embodiments, the computing unit further comprises a needle trajectory module which is configured to use as input the electrode configuration generated by the electrode module and to determine, for each of the electrodes, a percutaneous trajectory for a needle (specifically for a needle electrode end-point) for use in an ablation planning.

According to some embodiments, refinements, or variants of embodiments, the soft tissue emulation system is implemented as a portable user equipment. The fast performance of the emulation system of the invention opens in particular the possibility that the representation of the evolution of soft tissue over time can be performed in real-time on a cell phone or a tablet. Thus, according to the fourth aspect, the invention also provides a cell phone or a tablet comprising executable program code configured to, when executed, perform the method according to the second aspect of the present invention.

Although here, in the foregoing and also in the following, some functions are described as being performed by modules, it shall be understood that this does not necessarily mean that such modules are provided as entities separate from one another. In cases where one or more modules are provided as software, the modules may be implemented by program code sections or program code snippets, which may be distinct from one another but which may also be interwoven or integrated into one another.

Similarly, in cases where one or more modules are provided as hardware, the functions of one or more modules may be provided by one and the same hardware component, or the functions of several modules may be distributed over several hardware components, which need not necessarily correspond to the modules. Thus, any apparatus, system, method and so on which exhibits all of the features and functions ascribed to a specific module shall be understood to comprise, or implement, said module. In particular, it is a possibility that all modules are implemented by program code executed by the computing device, for example a server or a cloud computing platform.

The above embodiments and implementations can be combined with each other as desired, as far as this is reasonable.

Further scope of the applicability of the present method and apparatus will become apparent from the following figures, detailed description and claims. However, it should be understood that the detailed description and specific examples, while indicating preferred embodiments of the invention, are given by way of illustration only, since various changes and modifications within the spirit and scope of the invention will become apparent to those skilled in the art.

Aspects of the present disclosure will be better understood with reference to the following figures.

In the figures:

Fig. 1 is a schematic depiction of a computer-implemented soft tissue emulation system according to an embodiment of the present invention, preferably to be applied to cardiac tissue;

Fig. 2 is a block diagram showing an exemplary embodiment of a computer-implemented soft tissue emulation method;

Fig. 3 is a schematic illustration of the architecture of the artificial neural network of a cardiac tissue emulation system according to an embodiment of the present invention;

Fig. 4 is a schematic depiction of a computer-implemented soft tissue emulation system for hepatic tissue according to an embodiment of the present invention;

Fig. 5 is a block diagram showing an exemplary embodiment of a computer-implemented soft tissue emulation method for hepatic tissue under heat stimulation;

Fig. 6 is a schematic illustration of the architecture of the artificial neural network of a hepatic tissue emulation system according to an embodiment of the present invention;

Fig. 7 shows a possible flow chart for a computer-implemented patient-specific electrode determination method as part of a radio frequency ablation (RFA) planning according to an embodiment of the present invention;

Fig. 8 shows a flow chart describing a needle trajectory determining method for use in an RFA planning according to some embodiments of the present invention;

Fig. 9 shows a number of exemplary illustrations of some of the steps involved in the needle trajectory determining method described in Fig. 8;

Fig. 10 is a schematic block diagram illustrating a computer program product according to an embodiment of the third aspect of the present invention; and

Fig. 11 is a schematic block diagram illustrating a non-transitory computer-readable data storage medium according to an embodiment of the fourth aspect of the present invention.

Parts in the different figures that correspond to the same elements have been indicated with the same reference numerals.

The components in the drawings are not necessarily to scale, emphasis being placed instead upon clearly illustrating the principles of the present disclosure. Likewise, certain components can be shown in generalized or schematic form in the interest of clarity and conciseness. In some instances, well-known structures and devices are shown in block diagram form in order to avoid obscuring the concepts of the present invention.

The numeration of the steps in the methods are meant to ease their description. They do not necessarily imply a certain ordering of the steps. In particular, several steps may be performed concurrently.

The detailed description includes specific details for the purpose of providing a thorough understanding of the present invention. However, it will be apparent to those skilled in the art that the present invention may be practised without these specific details.

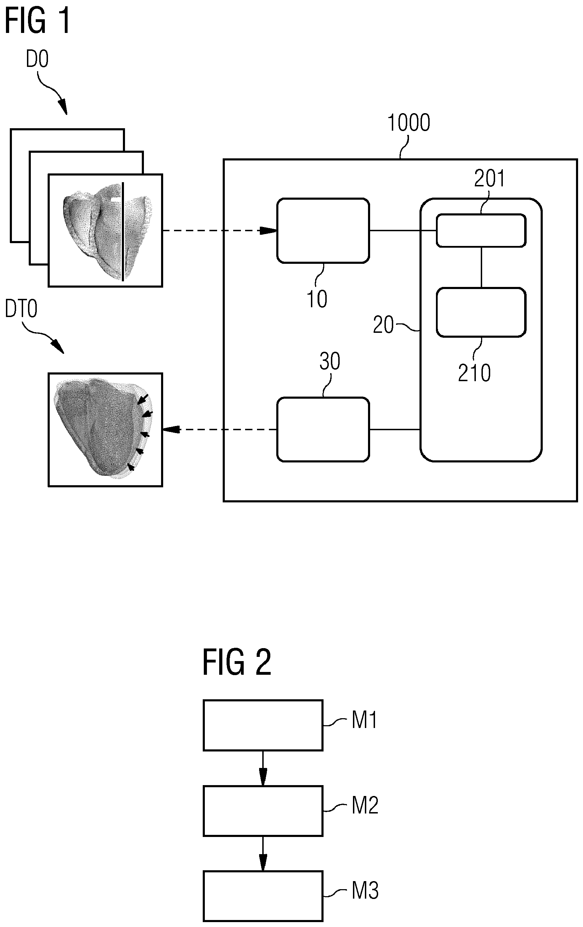

Fig. 1 shows a schematic depiction of a computer-implemented soft tissue emulation system 1000 according to an embodiment of the present invention. The soft tissue emulation system 1000 comprises an input interface 10, a computing unit 20 and an output interface 30.

The input interface 10 is configured to obtain imaging data D0 pertaining to soft tissue, e.g. from a Picture Archiving and Communication System (PACS) or directly from an imaging device. In the particular case of cardiac tissue emulation, the imaging data D0 comprises magnetic resonance imaging (MRI) data of cardiac tissue and/or electrocardiograms.

The computing unit 20 is configured to implement an artificial neural network, ANN 210, which is trained and adapted to generate a digital twin DT0 of the cardiac tissue at different times. This digital twin DT0 is obtained through the use of the obtained imaging data D0, from which the structural properties of the heart are inferred, and the use of a biophysical model, specifically an elastodynamics model, which is expressed as a differential equation which models the response of the cardiac tissue to electromechanical stimuli over time.

The ANN 210 is configured to implement a non-autoregressive method, such that the generation of the digital twin DT0 at one time is independent of the generation of the digital twin DT0 (or, more precisely, of its state) at another time.

The computing unit 20 may comprise a pre-processing module 201, which is configured to adapt the imaging data D0 for use by the ANN 210. The pre-processing module 201 is configured to perform segmentations and provide annotations of the imaging data D0. For cardiac tissue, in particular, the pre-processing module 201 is configured to apply a discretization of the imaging data D0 (after segmentation and annotation) using a tetrahedral mesh.

The output interface 30 is configured to output a representation DTO of the cardiac tissue over time based on the digital twin generated by the ANN 210.

Fig. 2 shows a block diagram showing an exemplary embodiment of a computer-implemented soft tissue emulation method, to be implemented preferably with the soft tissue emulation system 1000 of Fig. 1. In the following, cardiac tissue and the emulation of its response to electrical stimuli will be used as a working example. The scope of the invention is however not restricted to this particular example.

In a step M1, imaging data D0 of a soft tissue is obtained. In the particular case of cardiac tissue, this imaging data comprises MRI images and electrocardiograms.

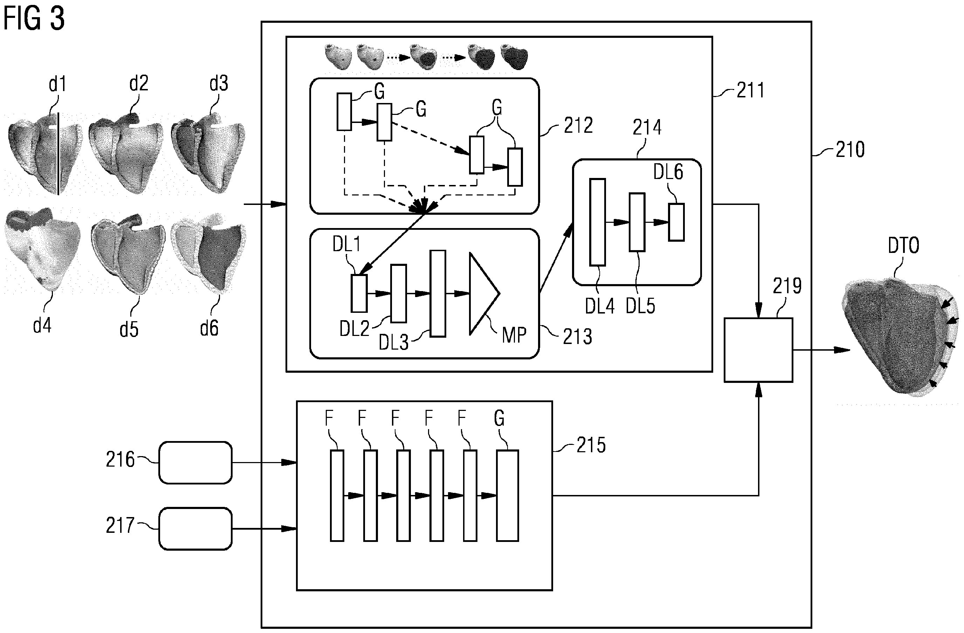

In a step M2, a digital twin of the cardiac tissue is generated at different points in time by using an artificial neural network 210, which is trained to learn the electrophysiological characteristics of the heart based on an elastodynamics model, in this case a differential equation. The ANN 210 is configured to solve the differential equation subject to a set of electromechanical stimuli with non-autoregressive methods (see Fig. 3 for details).

In a step M3, a representation DT0 of the cardiac tissue over time, i.e. its deformation under the electromechanical stimuli, is output, wherein the representation is based on the digital twin generated at different times.

Fig. 3 shows a schematic illustration of one option for the architecture of the artificial neural network 210 of the cardiac tissue emulation system 1000 according to Fig. 1, or for use in the method of Fig. 2.

The ANN 210 is based on an architecture which involves two convolutional neural networks structures, a branch network module 211 and a trunk network module 215.

The imaging data D0 obtained by the input interface 10 is used to infer geometric features of the heart by the branch network module 211. The imaging data D0 may have been segmented and provided with annotations, and then discretized using a tetrahedral mesh by e.g. the pre-processing module 201 of Fig. 1, where each vertex is assigned a set of geometric and electrophysiological features. The positions of the vertices are characterized with local 3D cylindrical coordinates (radius d1, height d2 and angle d3). This choice of coordinates facilitates that the ANN 210 learns about axial rotations of a heart and thus the training of the ANN 210 is achieved with less training data. Additionally, the relative positions of left and right ventricles d6, or endocardium and epicardium d5 are also included. Some vertices can also be characterized using categorical features, e.g. one may label vertices belonging to the endocardium or epicardium to better model the boundary conditions (i.e. blood pressure force on the endocardium and external force on epicardium simulating the interaction with the pericardium). The electrophysiological features ascribed to a vertex may be given either as local activation times d4 or, alternatively, as a sequence of potentials, which can either be taken as a vector or as encoding computed by another neural network.

The vertex structure of the tetrahedral mesh can be mathematically described as a graph. The ANN 210 architecture of Fig. 3 is that of a graph convolutional neural network, which are best suited to extract information from graph structured data, such as a set of vertices arranged in a 3D space. In particular, the architecture proposed in Fig. 3 uses a local feature extractor module 212, a global feature extractor module 213 and a prediction network module 214.

The local feature extractor module 212 is based on a cascade of between 10 and 30, here e.g. 20 GraphSAGE layers G with e.g. 32 units each. GraphSAGE layers G are fully connected layers that act on the local neighborhood of each each vertex independently, but are able to share learnable feature information between the vertices. Information on a neighborhood of a vertex is aggregated using the mean and then transferred from one layer G to the next layer G with a non-linear activation function. This concatenation of the input features together with the output features of each layer G is stored as a local feature matrix, which is processed by the global feature extractor module 213. A series of between 2 and 5 dense or fully connected layers, here e.g. 3 dense layers DL1, DL2 and DL3 with 256, 512 and 1024 nodes, respectively, is applied before aggregating the information via max pooling MP.

Local and global features are then concatenated per vertex and further processed by the prediction network module 214, which comprises a number of dense or fully connected layers, here e.g. 3 dense layers DL4, DL5 and DL6 with 512, 256 and 150 units, respectively. Through the combined action of the local feature extractor 212, the global feature extractor 213 and the prediction network 214, the branch network module 211 learns the geometry of the heart, i.e. it infers a latent feature vector for each vertex.

In order to learn about the evolution with time of the learned geometry one may use the solution of an elastomechanics equation, e.g. the second order differential equation

$$M\ddot{u} + C\dot{u} + Ku = F$$

where u is the local displacement vector of the cardiac tissue, $\dot{u}$ the velocity vector and $\ddot{u}$ the acceleration vector. M, C, and K are matrices encoding constitutive information (i.e. information about the material properties of the cardiac tissue). F is the (electric) force applied to the cardiac tissue derived from electric potentials.

The local displacement vector u is the solution operator to be solved using non-autoregressive methods. This is found by running the trunk network module 215, whose input are the times 216 (or query times), for which the solution operator is computed. Additionally, the trunk network module 215 may be configured to receive, as further input, model parameters 217 that shape or define the matrices M, C, and K.

The input features are then transformed by a series of fully connected layers, here e.g. 5 fully connected layers F with 100 units each and one GraphSAGE layer G with 150 units. The output of the trunk network module 215 is a latent code matching in dimension with the latent code of each vertex obtained by the branch network module 211. A dot-product module 219 is configured to perform a dot product between the latent codes generated by the branch network module 211 and the trunk network module 215, thereby obtaining the local displacement vectors u of the cardiac tissue at the different query times 216. The local displacement vectors over the full geometry of the cardiac tissue define the digital twin 218 for each query time.

The ANN 210 is preferably trained using a supervised approach. To this end, a large set of simulations is generated using a cardiac computational model of electromechanics. Specifically, a sampling module (not shown in the figure) may be configured to sample varying geometries from a statistical shape model. From the set of geometries, a simulation module may be configured to simulate different physiologically plausible cardiac activations using a model of cardiac electrophysiology. The set of reference simulations can be generated with conventional methods, e.g. with the total Lagrangian explicit dynamics (TLED) algorithm, where the material parameters can be additionally varied. Finally, the network is fitted using, as the objective function, a mean squared error loss and, e.g. the Adam optimizer.

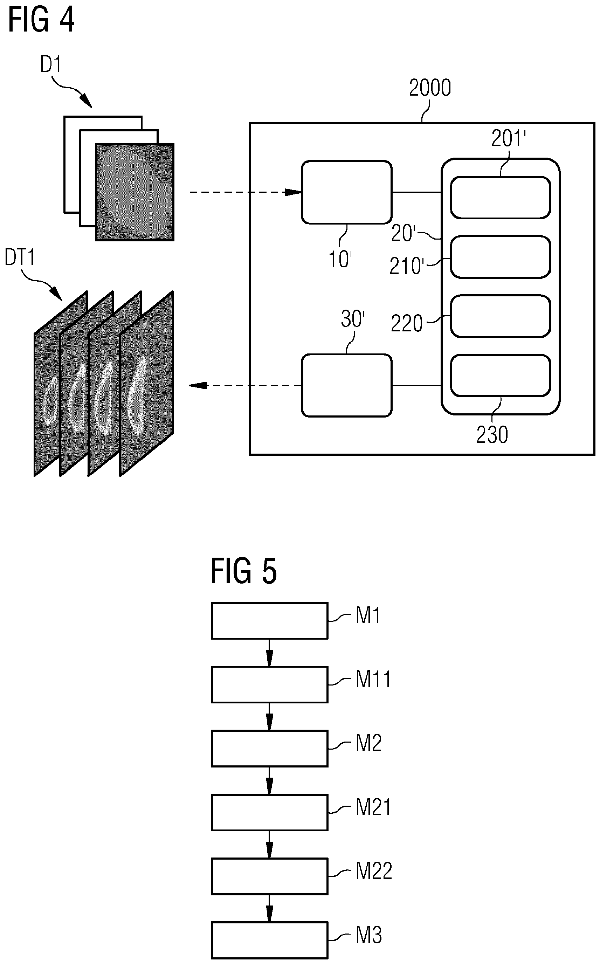

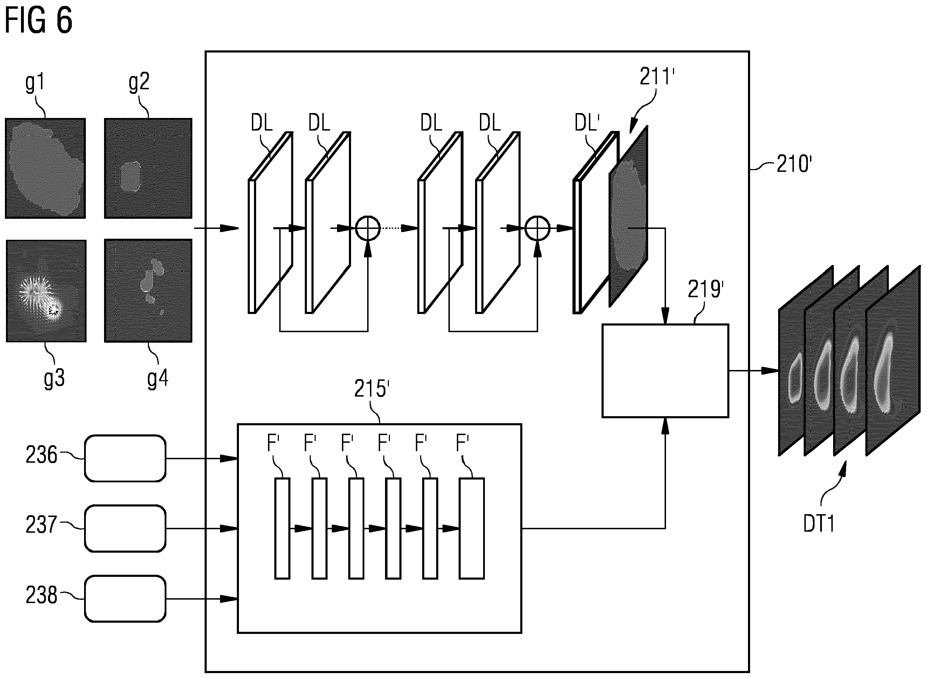

Fig. 4 shows a schematic depiction of a computer-implemented soft tissue emulation system 2000 for hepatic tissue according to an embodiment of the present invention. Details of the system 2000 will also be explained with respect to Fig. 6.

The system 2000 can contribute to a radio frequency ablation (RFA) planning, in which hepatic tumors are treated with a heating source, which is provided by an electrode mounted at the tip of an ablation needle, which is inserted in the body through a percutaneous path.

The hepatic tissue emulation system 2000 comprises an input interface 10', a computing unit 20' and an output interface 30'.

The input interface 10' is configured to obtain imaging data D1 from hepatic tissue, e.g., from a PACS or directly from an imaging device. For the liver, in particular, imaging data D1 may comprise computer tomography (CT) scans of the hepatic tissue, which provide in particular information about the configuration of the vessels in the liver together with the presence of tumors.

The computing unit 20' is configured to implement an artificial neural network, ANN 210', an electrode module 220 and a needle trajectory module 230.

The computing unit 20' may also comprise a pre-processing module 201', which is configured to adapt the imaging data D1 for use by the ANN 210'. The pre-processing module 201' is configured to perform segmentations and provide annotations of the imaging data D1. For hepatic tissue, in particular, the pre-processing module 201' is configured to apply a discretization of the imaging data D1 (after segmentation and annotation) using an Euclidean grid structure of voxels.

The ANN 210' is trained and adapted to generate a digital twin DT1 of the hepatic tissue at different times. This digital twin DT1 is obtained through the use of the obtained imaging data D1 (after segmentation and/or annotations and/or discretization has been performed by the pre-processing module 201'), from which the structural properties of the liver are inferred, and the use of at least an advecto-diffusion model, which is expressed as a differential equation which models the temperature of the hepatic tissue under the action of heat sources over time.

The ANN 210' is configured to implement a non-autoregressive method, such that the generation of the digital twin DT1 of the liver tissue at one time is independent of the generation of the digital twin DT1 of the liver tissue (or, more precisely, of its state) at another time.

The electrode module 220 is configured to use the information about the presence of tumors in the hepatic tissue as input, and generate as output a corresponding configuration of electrodes for the ablation planning, characterized at least by their location, their radius, and/or the time during which they are active.

The needle trajectory module 230 is configured to use as input the electrode configuration generated by the electrode module 220 and determine, for each of the heat sources, a percutaneous trajectory for a needle for use in an ablation planning and/or an ablation procedure.

The output interface 30' is configured to output a representation of the hepatic tissue over time based on the digital twin DT1 of the liver tissue generated by the ANN 210'. This representation could be, e.g. a 3D rendered image on a screen, which can be viewed from all angles, where the temperature distribution of the liver tissue or parts thereof is shown at different time points.

Fig. 5 is a block diagram showing an exemplary embodiment of a computer-implemented hepatic tissue emulation method, to be implemented preferably with the soft tissue emulation system 2000 of Fig. 4, for application to a radio frequency ablation (RFA) planning.

In a step M1, imaging data D1 is obtained. This imaging data D1 may comprise CT images of the hepatic tissue but is not restricted to it. Information about the torso, spine, ribs and organs that could be in the needle trajectory planning of the ablation procedure may also be contained in the obtained imaging data D1.

In a step M11, information about the presence of tumors in the hepatic tissue, specifically information about their position, size and shape, is obtained. This information may be extracted from the same imaging data D1, in particular from the CT images.

In a step M2, a digital twin DT1 of the hepatic tissue (including vessels and tumors) is generated at different points in time by using an artificial neural network 210', which is trained to learn the response or reaction of the hepatic tissue to heat sources (such as an electrode at the tip of a needle in an ablation procedure) over time based on an advection-diffusion model that takes into account the position of vessels in the liver. The ANN 210' is configured to solve the differential equation subject to the presence of a set of heat stimuli (e.g. electrodes) with non-autoregressive methods (see Fig. 6 for details on the ANN 210' architecture).

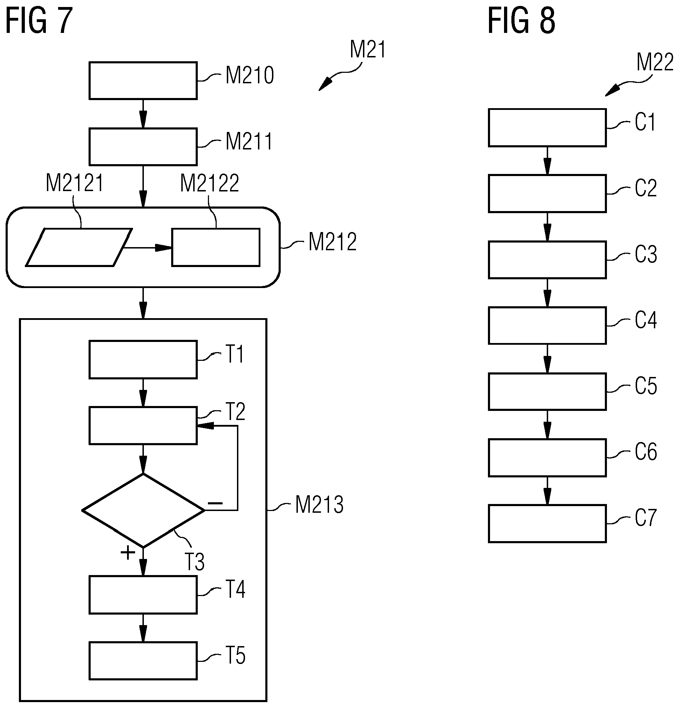

In a step M21, a set of electrodes characterized at least by their location, their radius and/or the time during which they are active is determined. This determination is based on the digital twin DT1 of the hepatic tissue and the acquired information about the presence of tumors. The determination of the electrode configuration is detailed in the description corresponding to Fig. 7.

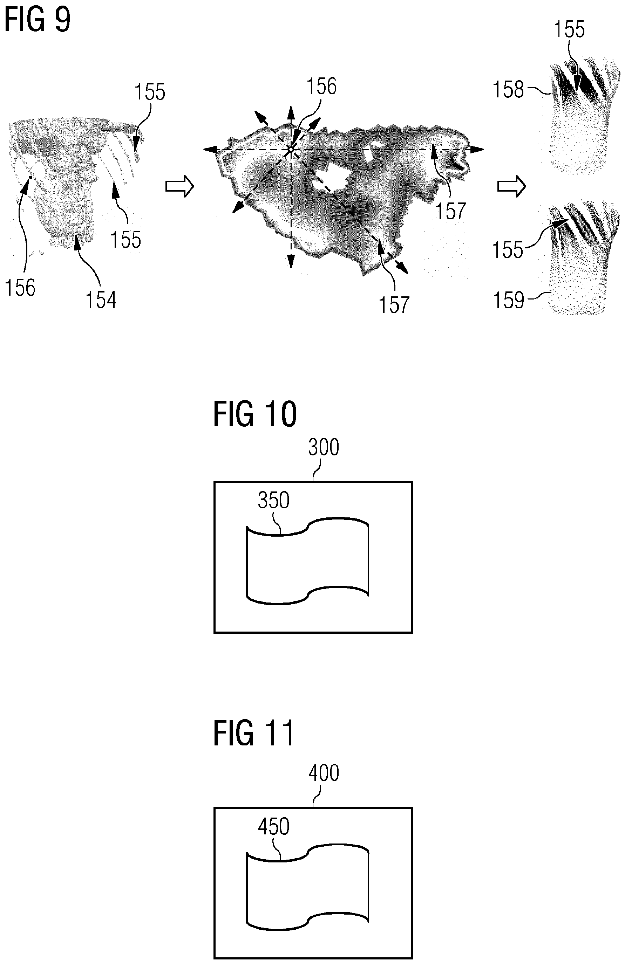

In another step M22, for each heat source determined in step M21, a percutaneous trajectory for a needle for use in an ablation procedure is determined. One possible method to determine the needle trajectory is specified in the description corresponding to Figs. 8 and 9.

In a step M3, a representation of the hepatic tissue over time, i.e. its reaction under the action of the heat sources, is output, wherein the representation is based on the digital twin DT1 generated at different times. This representation could be, e.g. a 3D rendered image on a screen, which can be viewed from all angles, where the temperature distribution of the liver tissue or parts thereof is shown at different time points.

Fig. 6 shows a schematic illustration of the architecture of the artificial neural network, ANN 210' of a hepatic tissue emulation system 2000 according to an embodiment of the present invention.

The ANN 210' is based on an architecture which involves two convolutional neural network structures, a branch network module 211' and a trunk network module 215'.

Input to the branch network module 211' comprises representations of the liver geometry g1 and its corresponding vessels g4, e.g. as binary masks, as well as information about the targeted electrode source positions g2, and the perfusion g3 (or blood velocity field, or advection field). Information about the liver geometry g1 and the vessels g4 may be obtained from CT images, which may have been segmented and provided with annotations (using for instance machine-learning based methods), and then discretized in voxels on an Euclidean grid by, e.g., the pre-processing module 201' of Fig. 3. The pre-processing module 201' may also be configured to compute the perfusion g3 using a fluid dynamics solver and to represent the electrode source positions g2 by a binary mask holding one or more spherical areas.

The information comprised in g1-g4 is passed through a series of convolutional layers DL (e.g. a number of ResNet blocks with 14 convolutional layers DL and 64 filters per layer) followed by a final layer DL'. A skip connection is added to each layer DL of a ResNet block, so that the input and output of a layer DL get added before a leaky rectified linear unit as activation function is applied. The output of the branch network module 211' comprises a latent encoding corresponding to each of the voxels of the input volume. The geometry and blood velocity field are encoded, e.g., as a volume of 150-dimensional latent codes.

For an RFA planning, one may determine the temperature distribution of the hepatic tissue over time as a solution of an advection-diffusion differential equation given by

$$\rho c_{ti} \frac{\partial T}{\partial t} = Q + \nabla \cdot (d\nabla T) \begin{cases} +R(T_{b0} - T) \\ -\alpha_v \rho_b c_b \, \boldsymbol{v} \cdot \nabla T \end{cases}$$

which describes the evolution of temperature with time for hepatic tissue, where the first line applies if the voxel belongs to a vessel, and the second line applies otherwise.

In the equation above, T is the temperature, t the time, Q the heat source, d the (potentially inhomogeneous) heat conductivity of the hepatic tissue, R the so-called reactive scale coefficient, $T_{b0}$ the blood temperature, $\alpha_v$ the advective scaling, $v$ the velocity field, $\rho$ and $\rho_b$ the tissue and blood densities, respectively, and $c_{ti}$ and $c_b$ the tissue and blood heat capacities, respectively.

The trunk network module 215' is used to query the solution of the advection-diffusion differential equation at specific points in time 236 as well as for a specific heating duration 237 and a given time delay 238. These quantities are processed by a cascade of fully connected layers F' (e.g. 5 fully connected layers with 100 units each and a final layer with 150 units) to obtain a latent encoding matching the dimension of a single voxel, as given by the branch network module 211'. The two latent codes can be combined in a dot-product module 219' in order to obtain a digital twin DT1, which represents the temperature distribution of the hepatic tissue at times 236 when heat sources are applied to it.

The ANN 210' can be trained either with 3D temperature data of livers recorded during RFA procedures or, alternatively, with synthetic simulations of real patient geometries, e.g. with the lattice Boltzmann method. By using the latter method, large training data pools can be easily obtained.

Fig. 7 shows a possible flow chart for a computer-implemented patient-specific electrode determination method M21 as part of a radio frequency ablation (RFA) planning according to an embodiment of the present invention. The electrode determination method M21 comprises different steps. The different steps of the method M21 can be performed by the electrode module 220 depicted in Fig. 4.

In a step M210, the information gathered in steps M1 and M11 of Fig. 5, namely imaging data D1 such as CT images and other clinical information relevant for the RFA procedure, e.g., additional diseases and medication of a patient, together with segmentations of the liver (including tumors and vessels) and information about other critical structures (e.g. torso, ribs, surrounding organs, etc.) is used to set a number of geometric constraints that restrict the positions of the ablation zones.

In another step M211, a calibration of the parameters of the advection-diffusion equation is performed. This step may be performed, e.g., by a calibration submodule of the electrode module 220. The calibration submodule is configured to find a set of model parameters specific to the physiology of a particular patient. In one embodiment, the patient-specific values can be restricted to the heat conductivities, the tissue density, the perfusion (determined from the position of the vessels and their irrigation) and/or the blood velocity. The model can be initialized with nominal parameters, which are subsequently optimized by using the previously mentioned preoperatively acquired imaging data D1 (e.g. CT images and/or clinical information). In another embodiment, the optimization from pre-operative information may comprise the mapping of image intensities to spatially varying heat conductivities and tissue densities using a simplified rule-based model, i.e. by hand-crafting a linear mapping of image intensities to a reasonable parameter range. Alternatively, the calibration submodule may be set up such that population data is used to find a better set of parameters, grouping for instance patients with fatty liver disease into one class and prescribing a higher heat conductivity and lower tissue density compared to nominal healthy tissue.

In some embodiments, the calibration submodule can be set up such that spatially-varying perfusion and blood velocity are derived from perfusion imaging. In other embodiments, the calibration submodule may be configured to implement a fluid-dynamics computational model adapted to the patient.

According to some embodiments, another step M212 is provided, wherein the calibration submodule is further adapted to use intra-operative information, which can be acquired in a step M2121. This additional information may comprise data about the apparent temperature recorded over time during an RFA procedure on the same patient. For instance, the temperature profile measured at the electrode tips during the ablation of a single tumor may be retrieved by the calibration submodule and used to fine-tune, e.g., the heat conduction coefficient of the hepatic tissue. Similarly, additional postablation CT images of a single tumor can be obtained by the calibration submodule to better determine the ablation zone extent. The information obtained in M2121 can be, if needed, automatically segmented in a further step M2122. Conventional optimizers can be deployed to finetune the model parameters such that the discrepancy between the simulated and the real ablation zones is minimized. Given the speed performance that is achieved with the emulation system 2000 of the invention, the model calibration step M212 can be actually performed in real-time, i.e. during the actual RFA procedure.

Once the model parameters have been tuned to patient-specific values, an electrode determination can be performed in a step M213. This step consists in finding the optimal electrode configuration, i.e. one that achieves full coverage of the treatment zone (i.e. tumor and safety margin) while minimizing damage to surrounding tissue. Specifically, this step M213 seeks to find the optimal number of electrodes as well as their locations and the respective electrode radius. Important parameters such as the optimized heating duration are also taken into account. The step M213 comprises a number of steps.

In a step T1, a number of constraints are selected, such as minimum and maximum number of electrodes, range and number of heating points and duration of the heating at each point. This selection may be performed by a clinician, e.g. according to experience, based on patient-specific information and/or based on medical protocols.

In a step T2, the computational model is run, starting with the minimal number of electrodes. The model searches a solution of the differential equation for each ablation zone by varying the heating duration and the electrode radii. For each simulation the coverage of the treatment zone is computed as well as the extent of the over-ablation zone, i.e. the voxels outside the treatment zone but inside the ablation zone.

In a further step T3, the results, which can be e.g. stored in a matrix, are filtered to determine a configuration that achieves full coverage with the least damage to surrounding tissue.

If such a configuration is found, then the system proceeds to a step T4, in which an electrode configuration for an ablation planning is released. If no solution is found in step T3, the model is run again by increasing the number of electrodes, and the preceding testing procedure is applied with the updated configuration. The process is iterated until a solution is found, where a solution means a successful ablation, where the tumors can be eliminated by inflicting minimal damage to the surrounding tissue.

In some of the embodiments, a validation step T5 is performed after an optimal solution has been found. The validation consists in running a different numerical solver with physical and accuracy guarantees, e.g., the Lattice Boltzmann method. The solution leading to the RFA planning is validated if the learned and the numerically solved ablation zone estimates do not show a discrepancy beyond a certain predefined error margin, which can, e.g., be assessed by a clinician.

In cases where an improved computing performance is available and in which computing time is not a limitation, the procedure described in steps T1-T3 can be varied. For instance, one may test the full scope of the parameter space and determine the global optimal solution in a single step.

For improved computational performance, one may use state-of-the-art hardware and software accelerations, e.g. by the use of graphics processing units and efficient parallelization techniques that enable the testing of multiple hypotheses at the same time. Depending on the infrastructure, the optimization can be performed on a high-performance computing (HPC) cluster.

For patients with multiple tumors, the steps of intra-operative data acquisition and model calibration or refinement can be performed multiple times. The physician can hereby rank the tumors according to the difficulty in ablating them. By iteratively applying the method M212 from easy to difficult tumors it is expected that the planning for very difficult tumors becomes much more accurate.

Fig. 8 shows a flow chart describing a needle trajectory determining method M22 for use in an RFA planning according to some embodiments of the present invention. A needle trajectory determining method M22 aims at finding a suitable entry point on the patient's torso as well as finding an optimal path to the ablation zones. The needle path typically needs to fulfill at least the following constraints:

> **1)** Avoiding organs and other structures that could be damaged (e.g. vessels, lungs, spine or ribs)
> **2)** The ablation target has to adapt to the length of the needle.
> **3)** The liver capsule has to be accessed at an angle not smaller than 20°
> **4)** The path should traverse a minimum of 5mm of liver tissue.

The needle trajectory determining method M22 comprises a number of steps. This steps may be performed by the needle trajectory module 230 depicted in Fig. 4.

In a step C1, taking as input the fully segmented CT images from the ribs, spine, organs, and/or vessels of the patient, as well as information about the presence of tumors, levelset representations in the form of signed distance fields are computed for each voxel. This computation can be performed by a signed-distance submodule of the needle trajectory module 230. The following levelset representations are computed:

> (a) Signed distance field within liver
> (b) Signed distance field to organs that can be damaged
> (c) Signed distance field to vessels
> (d) Signed distance field to ribs
> (e) Distance field to targeted electrode endpoint

In addition, binary mask denoting the liver and the torso are used.

Negative values hereby correspond to voxels inside the liver, ribs, vessels, etc., while positive values denote voxels outside of these structures. Consequently, ray-triangle intersections can be replaced by simply picking up the values at discrete locations along a ray's path, which can be massively parallelized on graphics processing units (GPUs). The accuracy is thereby only impacted by the underlying image resolution and the accuracy of the segmentation process.

In a step C2, a ray-tracing algorithm, implemented by, e.g., an algorithm submodule of the needle trajectory module 230, is used to determine a set of possible target points from the voxels inside the treatment zone. This involves the positioning of a pre-computed set of rays (e.g. using the Fibonacci lattice sphere algorithm) at the targeted electrode endpoint. The number of rays (in the range of 10-100.000) can be adapted according to the specific speed and hardware constraints.

In a step C3, sampling locations along the ray and through the volume are computed for each ray.

In a step C4, values of the levelset representations and binary masks collected in step C1 are sampled using e.g. tri-linear interpolation for all sampling locations.

In a step C5, the minimum and maximum values of the sample values corresponding to each ray are computed. Invalid rays, i.e. those not fulfilling the constraints 1) to 4) mentioned above, can thus be filtered out applying the following checks:

> (a) the minimum distance to critical structures (organs, vessels, ribs) is not negative, meaning that the ray does not cross the critical structure. A safety margin can be applied by checking for a minimum distance greater than a predetermined threshold. Separate threshold can be defined for the organs, vessels, and the ribs;

> (b) the largest distance to the source location is smaller than the length of the available needle;

> (c) the angle between the ray and the liver capsule is greater than 20°; and

> (d) the penetration distance within the liver is larger than 5mm.

In another step C6, possible candidates for the skin entry points are determined, taking as input the sampled values of the torso binary mask that are right outside of the torso.

According to some embodiments, in a step C7 the ray trajectories extending all the way from the entry points to the targeted electrode positions can be visualized in a 3D graphical user interface (GUI).

Fig. 9 shows a number of exemplary illustrations of some of the steps involved in the needle trajectory determining method M22 described in Fig. 8. On the left panel an example of a segmentation of CT images of a patient is shown. From the picture one can identify the spine 154, the ribs 155 as well as an electrode endpoint 156 on the liver.

The central panel of Fig. 9 shows a cross section of the liver, with the electrode endpoint 156 and a number of rays 157 generated, e.g., with the ray-tracing algorithm described in step C2 of Fig. 8. The picture shows one particular levelset representation, namely the signed distance field from the electrode endpoint 156 to blood vessels.

The two pictures on the right panel of Fig. 9 show two more levelset representations, namely the distance field to targeted electrode endpoint (picture 158 on the top) and the minimum distance to organs (picture 159 on the bottom). The white traces in both pictures 158 and 159 are the ribs 155.

Fig. 10 shows a schematic block diagram illustrating a computer program product 300 according to an embodiment of the third aspect of the present invention. The computer program product 300 comprises executable program code 350 configured to, when executed, perform the method according to any embodiment of the second aspect of the present invention, in particular as it has been described with respect to the preceding figures.

Fig. 11 shows a schematic block diagram illustrating a non-transitory computer-readable data storage medium 400 according to an embodiment of the fourth aspect of the present invention. The data storage medium 400 comprises executable program code 450 configured to, when executed, perform the method according to any embodiment of the second aspect of the present invention, in particular as it has been described with respect to the preceding figures.

The non-transient computer-readable data storage medium may comprise, or consist of, any type of computer memory, in particular semiconductor memory such as a solid-state memory. The data storage medium may also comprise, or consist of, a CD, a DVD, a Blu-Ray-Disc, an USB memory stick or the like.

The previous description of the disclosed embodiments are merely examples of possible implementations, which are provided to enable any person skilled in the art to make or use the present invention. Various variations and modifications of these embodiments will be readily apparent to those skilled in the art, and the generic principles defined herein may be applied to other embodiments without departing from the spirit or scope of the present disclosure. Thus, the present invention is not intended to be limited to the embodiments shown herein but it is to be accorded the widest scope consistent with the principles and novel features disclosed herein. Therefore, the present invention is not to be limited except in accordance with the following claims.

**Claims**

1. A soft tissue emulation system (1000, 2000), comprising:

   an input interface (10, 10'), configured to obtain imaging data (D0, D1) of the soft tissue;
   a computing unit (20, 20'), configured to implement an artificial neural network (210, 210'), which is adapted to generate, using the obtained imaging data (D0, D1) as input and a biophysical model of the soft tissue, a digital twin (DT0, DT1) of the soft tissue at different times (216, 236), wherein the biophysical model describes the response of the soft tissue to thermal and/or electromechanical stimuli over time, and wherein the generation of the digital twin (DT0, DT1) at one time (216, 236) is independent of the generation of the digital twin (DT0, DT1) at another time (216, 236); and
   an output interface (30, 30'), configured to output a representation of the soft tissue over time based on the digital twin (DT0, DT1) generated by the artificial neural network (210, 210').

2. The system (1000, 2000) according to claim 1, wherein the artificial neural network (210, 210') comprises a branch network module (211, 211'), which implements a first convolutional neural network configured to infer structural information of the soft tissue, and a trunk network module (215, 215'), which implements a second convolutional neural network configured to generate a solution to the biophysical model at different times (216, 236).

3. The system (1000) according to any of the previous claims, wherein the system (1000) is configured to emulate the electromechanical properties of cardiac tissue.

4. The system (1000) according to claim 3, wherein the biophysical model is based on a second order ordinary differential equation describing the deformation of cardiac tissue in response to at least one electrophysiological information.

5. The system (1000) according to claim 4, wherein the electrophysiological information comprises a set of local activation times (d4) and/or a sequence of electric potentials.

6. The system (1000) according to claim 2 in combination with one of the claims 3 to 5, wherein the structural information of the soft tissue comprises a tetrahedral mesh, with each vertex **characterized by** a set of local cylindrical coordinates (d1, d2, d3) and/or a distance between endocardium and epicardium (d5), and/or an indication whether a vertex belongs to the endocardium or to the epicardium (d6).

7. The system (2000) according to any of the previous claims, wherein the system (2000) is configured to emulate the local temperature distribution of the soft tissue when stimulated by an external heat source.

8. The system (2000) according to claim 2, wherein the structural information of the soft tissue is **characterized by** at least one of the following physiological properties: density, heat conductivity, location of the blood vessels and blood flow rate.

9. The system (2000) according to claim 8, wherein the soft tissue is hepatic tissue, wherein the imaging data (D1) comprises information about the presence of tumors in the hepatic tissue, and wherein the computing unit (20') further comprises an electrode module (220), configured to use the information about the presence of tumors in the hepatic tissue as input, and generate as output a corresponding electrode configuration for an ablation planning, characterized at least by their location, their radius, and/or the time during which they are active.

10. The system (2000) according to claim 9, wherein the computing unit (20') further comprises a needle trajectory module (230), which is configured to use as input the electrode configuration generated by the electrode module (220) and determine, for each of the electrodes, a percutaneous trajectory for a needle for use in an ablation planning.

11. The system (1000, 2000) according to any of the previous claims, implemented as a portable user equipment.

12. A computer-implemented soft tissue emulation method, preferably to be implemented with the soft tissue emulation system (1000, 2000) according to any of claims 1 to 11, comprising the following steps:

    obtaining (M1) imaging data (D0, D1) of a soft tissue;
    generating (M2), using a biophysical model of the soft tissue and an artificial neural network (210, 210'), configured and trained to receive the acquired imaging data (D0, D1) as input, a digital twin (DT0, DT1) of the soft tissue at different times (216, 236), wherein the biophysical model simulates the response of the soft tissue to thermal and/or electromechanical stimuli over time, and wherein the generating of the digital twin (DT0, DT1) at one time (216, 236) is independent of the generating of the digital twin (DT0, DT1) at another time (216, 236); and
    outputting (M3) a representation of the soft tissue over time based on the digital twin (DT0, DT1) generated at the different times (216, 236).

13. The computer-implemented method of claim 12, wherein the soft tissue is hepatic tissue, the method further comprising:

    acquiring (M11) information about the presence of tumors in hepatic tissue;
    determining (M21), based on the digital twin (DT1) of the hepatic tissue and the acquired information about the presence of tumors, a corresponding electrode configuration characterized at least by the location of the electrodes, their radius and/or the time during which they are active; and
    determining (M22), for each of the electrodes, a percutaneous trajectory for a needle for use in an ablation planning.

14. A computer program product (300) comprising executable program code (350), which is configured, when executed, to perform the computer-implemented method according to claim 12 and/or claim 13.

15. A non-transient computer-readable data storage medium (400) comprising executable program code (450), which is configured, when executed, to perform the computer-implemented method according to claim 12 and/or claim 13.

**Amended claims in accordance with Rule 137(2) EPC.**

1. A soft tissue emulation system (1000, 2000), wherein the soft tissue is hepatic tissue, comprising:

    an input interface (10, 10'), configured to obtain imaging data (D0, D1) of the soft tissue and to acquire information about the presence of tumors in hepatic tissue;
    a computing unit (20, 20'), configured to implement an artificial neural network (210, 210'), which is adapted to generate, using the obtained imaging data (D0, D1) as input and a biophysical model of the soft tissue, a digital twin (DT0, DT1) of the soft tissue at different times (216, 236), wherein the biophysical model describes the response of the soft tissue to thermal and/or electromechanical stimuli over time, and wherein the generation of the digital twin (DT0, DT1) at one time (216, 236) is independent of the generation of the digital twin (DT0, DT1) at another time (216, 236), and

        configured to determine, based on the digital twin (DT1) of the hepatic tissue and the acquired information about the presence of tumors, a corresponding electrode configuration characterized at least by the location of the electrodes, their radius and/or the time during which they are active, and configured to determine, for each of the electrodes, a percutaneous trajectory for a needle for use in an ablation planning; and

    an output interface (30, 30'), configured to output a representation of the soft tissue over time based on the digital twin (DT0, DT1) generated by the artificial neural network (210, 210').

2. The system (1000, 2000) according to claim 1, wherein the artificial neural network (210, 210') comprises a branch network module (211, 211'), which implements a first convolutional neural network configured to infer structural information of the soft tissue, and a trunk network module (215, 215'), which implements a second convolutional neural network configured to generate a solution to the biophysical model at different times (216, 236).

3. The system (2000) according to any of the previous claims, wherein the system (2000) is configured to emulate the local temperature distribution of the soft tissue when stimulated by an external heat source.

4. The system (2000) according to claim 2, wherein the structural information of the soft tissue is **characterized by** at least one of the following physiological properties: density, heat conductivity, location of the blood vessels and blood flow rate.

5. The system (1000, 2000) according to any of the previous claims, implemented as a portable user equipment.

6. A computer-implemented soft tissue emulation method, wherein the soft tissue is hepatic tissue, preferably to be implemented with the soft tissue emulation system (1000, 2000) according to any of claims 1 to 5, comprising the following steps:

   obtaining (M1) imaging data (D0, D1) of a soft tissue;
   acquiring (M11) information about the presence of tumors in hepatic tissue;
   generating (M2), using a biophysical model of the soft tissue and an artificial neural network (210, 210'), configured and trained to receive the acquired imaging data (D0, D1) as input, a digital twin (DTO, DT1) of the soft tissue at different times (216, 236), wherein the biophysical model simulates the response of the soft tissue to thermal and/or electromechanical stimuli over time, and wherein the generating of the digital twin (DTO, DT1) at one time (216, 236) is independent of the generating of the digital twin (DTO, DT1) at another time (216, 236);
   determining (M21), based on the digital twin (DT1) of the hepatic tissue and the acquired information about the presence of tumors, a corresponding electrode configuration characterized at least by the location of the electrodes, their radius and/or the time during which they are active;
   determining (M22), for each of the electrodes, a percutaneous trajectory for a needle for use in an ablation planning; and
   outputting (M3) a representation of the soft tissue over time based on the digital twin (DTO, DT1) generated at the different times (216, 236).

7. A computer-implemented soft tissue emulation method for emulating the electromechanical properties of cardiac tissue , comprising the following steps:

   obtaining (M1) imaging data (D0, D1) of a soft tissue;
   generating (M2), using a biophysical model of the soft tissue and an artificial neural network (210, 210'), configured and trained to receive the acquired imaging data (D0, D1) as input, a digital twin (DTO, DT1) of the soft tissue at different times (216, 236), wherein the biophysical model simulates the response of the soft tissue to thermal and/or electromechanical stimuli over time, wherein the biophysical model is based on a second order ordinary differential equation describing the deformation of cardiac tissue in response to at least one electrophysiological information, wherein the electrophysiological information comprises a set of local activation times (d4) and/or a sequence of electric potentials comprising information about the space and time dependence of the electrical stimuli applied onto the cardiac tissue, and wherein the generating of the digital twin (DTO, DT1) at one time (216, 236) is independent of the generating of the digital twin (DTO, DT1) at another time (216, 236); and
outputting (M3) a representation of the soft tissue over time based on the digital twin (DTO, DT1) generated at the different times (216, 236).

8. The computer-implemented soft tissue emulation method of claim 7, wherein the artificial neural network (210, 210') comprises a branch network module (211, 211'), which implements a first convolutional neural network configured to infer structural information of the soft tissue, and a trunk network module (215, 215'), which implements a second convolutional neural network configured to generate a solution to the biophysical model at different times (216, 236).

9. The computer-implemented soft tissue emulation method of claim 8, wherein the structural information of the soft tissue comprises a tetrahedral mesh, with each vertex **characterized by** a set of local cylindrical coordinates (d1, d2, d3) and/or a distance between endocardium and epicardium (d5), and/or an indication whether a vertex belongs to the endocardium or to the epicardium (d6).

10. A computer program product (300) comprising executable program code (350), which is configured, when executed, to perform the computer-implemented method according to one of the claims 6 to 9.

11. A non-transient computer-readable data storage medium (400) comprising executable program code (450), which is configured, when executed, to perform the computer-implemented method according to one of the claims 6 to 9.

## FIG 1

D0

DT0

1000

201

10

20

30

210

## FIG 2

M1

M2

M3

FIG 3

FIG 4

FIG 5

FIG 6

FIG 7

M21

M210

M211

M2121    M2122

M212

T1

T2

M213

T3

+

T4

T5

FIG 8

M22

C1

C2

C3

C4

C5

C6

C7

FIG 9

FIG 10

FIG 11

# EP 4 345 829 A1

## EUROPEAN SEARCH REPORT

**Application Number**

EP 22 19 7988

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/325572 A1 (MANSI TOMMASO [US] ET AL) 24 October 2019 (2019-10-24) | 1-15 | INV. G16H20/40 |
| Y | * abstract; figures 1,4 * <br> * paragraph [0007] – paragraph [0008] * <br> * paragraph [0025] – paragraph [0028] * <br> * paragraph [0074] * <br> * paragraph [0017] * <br> * paragraph [0020] * | 3,7-9 | G16H30/40 <br> G16H50/50 |
| | ----- | | |
| Y | US 2021/151187 A1 (MANSI TOMMASO [US] ET AL) 20 May 2021 (2021-05-20) <br> * abstract; figure 1 * <br> * paragraph [0002] * <br> * paragraph [0006] – paragraph [0007] * <br> * paragraph [0017] * <br> * paragraph [0024] * <br> * paragraph [0027] * <br> * paragraph [0036] – paragraph [0038] * | 3,7-9 | |
| | ----- | | |
| X | SERMESANT MAXIME ET AL: "Applications of artificial intelligence in cardiovascular imaging", <br> NATURE REVIEWS CARDIOLOGY, NATURE PUBLISHING GROUP, GB, <br> vol. 18, no. 8, 12 March 2021 (2021-03-12) <br> , pages 600-609, XP037509639, <br> ISSN: 1759-5002, DOI: <br> 10.1038/S41569-021-00527-2 <br> [retrieved on 2021-03-12] <br> * left-hand column since beginning until fifth line on the right-hand column; page 600 * <br> * section "Biophysics-based AI"; page 604 * <br> * Section "Conclusions", third paragraph onwards; page 607 * | 1-15 | TECHNICAL FIELDS SEARCHED (IPC) <br><br> G16H |
| | ----- <br> -/-- | | |

The present search report has been drawn up for all claims

| Place of search | Date of completion of the search | Examiner |
|---|---|---|
| Munich | 7 March 2023 | Ricciardi, Maurizio |

EPO FORM 1503 03.82 (P04C01)

**page 1 of 2**

Europäisches
Patentamt

European
Patent Office

Office européen
des brevets

## EUROPEAN SEARCH REPORT

Application Number

EP 22 19 7988

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARK ALBER ET AL: "Multiscale modeling meets machine learning: What can we learn?", ARXIV.ORG, CORNELL UNIVERSITY LIBRARY, 201 OLIN LIBRARY CORNELL UNIVERSITY ITHACA, NY 14853, 27 November 2019 (2019-11-27), XP081540546, * abstract * * From last sentence on page 1 to second paragraph on page 2 * * Section "2.2 Applications and opportunities"; page 3 – page 4 * * Caption of figure 4 and paragraph immediately succeeding; page 8 * * Section "Physics-informed neural networks"; page 9 * | 1-15 | |

----

|  |  |  | TECHNICAL FIELDS SEARCHED (IPC) |
|---|---|---|---|

The present search report has been drawn up for all claims

| Place of search | Date of completion of the search | Examiner |
|---|---|---|
| Munich | 7 March 2023 | Ricciardi, Maurizio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 7988

This annex lists the patent family members relating to the patent documents cited in the above-mentioned European search report.
The members are as contained in the European Patent Office EDP file on
The European Patent Office is in no way liable for these particulars which are merely given for the purpose of information.

07-03-2023

| Patent document cited in search report | | Publication date | Patent family member(s) | | Publication date |
|---|---|---|---|---|---|
| US 2019325572 | A1 | 24-10-2019 | CN | 110400283 A | 01-11-2019 |
| | | | US | 2019325572 A1 | 24-10-2019 |
| US 2021151187 | A1 | 20-05-2021 | CN | 112640000 A | 09-04-2021 |
| | | | EP | 3824477 A1 | 26-05-2021 |
| | | | US | 2021151187 A1 | 20-05-2021 |
| | | | WO | 2020038873 A1 | 27-02-2020 |

EPO FORM P0459

For more details about this annex : see Official Journal of the European Patent Office, No. 12/82